(19) Europäisches Patentamt
European Patent Office
Office européen des brevets

(11) Numéro de publication : **0 286 536 B1**

(12)

# FASCICULE DE BREVET EUROPEEN

(45) Date de publication du fascicule du brevet :
**17.07.91 Bulletin 91/29**

(51) Int. Cl.⁵ : **B65G 29/00, G01N 35/04, G21F 7/00**

(21) Numéro de dépôt : **88400831.9**

(22) Date de dépôt : **06.04.88**

(54) **Transporteur rotatif à avance pas-à-pas et installation de prélèvement d'échantillons liquides comportant un tel transporteur.**

(30) Priorité : **09.04.87 FR 8704998**

(43) Date de publication de la demande :
**12.10.88 Bulletin 88/41**

(45) Mention de la délivrance du brevet :
**17.07.91 Bulletin 91/29**

(84) Etats contractants désignés :
**BE CH DE ES FR GB IT LI LU NL SE**

(56) Documents cités :
**EP-A- 0 101 192**
**US-A- 3 405 455**
**US-A- 3 686 960**
**US-A- 3 981 546**

(73) Titulaire : **COMPAGNIE GENERALE DES MATIERES NUCLEAIRES (COGEMA)**
**2, rue Paul Dautier B.P. 4**
**F-78141 Velizy-Villacoublay (FR)**

(72) Inventeur : **Chazot, Henri**
**10,rue de la Grange Rouge**
**F-30133 Les Angles (FR)**
Inventeur : **Cauquil, Gérard**
**Codolet**
**F-30200 Bagnols Sur Cèze (FR)**
Inventeur : **Muller, Jean-Paul**
**Les Hautes Planes**
**F-30200 Orsan (FR)**

(74) Mandataire : **Mongrédien, André et al**
**c/o BREVATOME 25, rue de Ponthieu**
**F-75008 Paris (FR)**

## Description

L'invention concerne un transporteur rotatif permettant de faire défiler pas-à-pas des récipients tels que des flacons devant un certain nombre de postes ·fixes. L'invention concerne également l'application d'un tel transporteur à la réalisation d'une installation automatisée de prélèvement d'échantillons liquides dans des flacons bouchés par des opercules, dans le but d'analyser ces échantillons.

La mise en oeuvre de certains procédés chimiques nécessite d'effectuer périodiquement des prélèvements des produits à différents stades du procédé, afin de les analyser pour contrôler leurs caractéristiques.

Dans l'industrie nucléaire, ces opérations de prélèvements sont compliquées par le fait qu'elles doivent être réalisées à l'intérieur de boîtes à gants ou d'enceintes blindées assurant la protection du personnel situé à l'extérieur.

Lorsque les prélèvements concernent des contrôles occasionnels réalisés en laboratoire, on utilise actuellement des pipettes de verre munies à leur partie supérieure d'un rodage sphérique relié à une seringue. Chaque prélèvement est effectué en plongeant la pointe de la pipette dans le liquide à prélever, puis en aspirant le liquide à l'aide de la seringue. Le contrôle de la quantité prélevée est obtenu au moyen d'une vis micrométrique adaptée sur la seringue.

Il est clair que la mise en oeuvre d'un tel appareillage requiert de nombreuses manutentions qui sont effectuées à distance par l'opérateur, par exemple à l'aide d'un télémanipulateur ou de gants invaginés dans la paroi de la cellule. Ainsi, aux manutentions nécessaires à l'exécution du prélèvement s'ajoute la nécessité de rincer la verrerie et d'essuyer l'extérieur de la pipette après chaque prélèvement. Cet appareillage est donc difficilement utilisable lorsque des prélèvements périodiques relativement rapprochés doivent être effectués sur une installation industrielle.

De plus, la verrerie doit être fréquemment renouvelée et l'appareillage doit comprendre des dispositifs permettant de déboucher et de reboucher les flacons.

Enfin, les volumes délivrés sont imprécis, car ils dépendent notamment de la vitesse de refoulement du liquide, de la viscosité de la solution, de la propreté de la verrerie et de l'ajustage du ménisque au trait de jauge de la pipette.

Par ailleurs, les transporteurs existant actuellement pour faire défiler automatiquement des échantillons devant différents postes ne sont pas utilisables à l'intérieur d'une boîte à gants ou d'une enceinte blindée comme l'exige l'industrie nucléaire.

En effet, les installations utilisées dans les cellules de confinement doivent pouvoir être dissociées en une partie proprement mécanique située à l'intérieur de la cellule et une partie de commande que l'on place à l'extérieur, ce qui n'est pas possible dans les transporteurs existants.

De plus, tous les transporteurs existants comprennent des mécanismes quine peuvent pas être utilisés en milieu radioactif sans subir de profondes modifications. Il en est de même des matériaux qui constituent ces mécanismes et qui sont également le plus souvent incompatibles avec un environnement radioactif.

Enfin, les transporteurs automatiques existants présentent des dimensions trop importantes pour permettre leur intégration dans une cellule dans laquelle le volume disponible est limité. De plus, leur coût est trop élevé, si l'on tient compte du fait qu'un installation implantée dans une cellule de confinement contenant des produits radioctifs doit pouvoir être remplacée périodiquement.

L'invention a précisément pour objet un transporteur rotatif à avance pas-à-pas dont la conception et la réalisation sont particulièrement adaptées à un fonctionnement à l'intérieur d'une cellule de confinement utilisée dans l'industrie nucléaire. En particulier, ce transporteur est conçu de telle sorte que la partie qui en assure la commande peut être dissociée du transporteur proprement dit et placée à l'extérieur de l'enceinte. De plus, ce transporteur peut fonctionner en milieu irradié et ses dimensions ainsi que son coût permettent son implantation en cellule et son remplacement périodique.

De façon plus précise, il est proposé conformément à l'invention un transporteur rotatif à avance pas-à-pas, caractérisé en ce qu'il comprend :

- un socle fixe pourvu d'une face supérieure horizontale sur laquelle débouche au moins un injecteur communiquant avec des moyens d'alimentation en gaz sous pression,
- un plateau tournant reposant par gravité sur la face supérieure du socle et coopérant avec cette face par des moyens de centrage du plateau autour d'un axe vertical fixe, ce plateau ayant un bord extérieur circulaire, une face supérieure pourvue de réceptacles répartis en couronne autour dudit axe et une face inférieure délimitant avec la face supérieure du socle une chambre centrale de sustentation, au moins un passage orienté selon une direction inclinée par rapport à un rayon passant par ledit axe étant formé dans le plateau pour relier la chambre centrale de sustentation et le bord extérieur du plateau, afin d'assurer une rotation de ce dernier lorsque les moyens d'alimentation sont actionnés, le plateau ayant un poids élevé tel que cette rotation soit stoppée instantanément lorsque les moyens d'alimentation sont arrêtés,
- des moyens de détection à distance de la présence d'un réceptacle dans une position angulaire donnée autour dudit axe,
- des moyens de commande sensibles à des signaux délivrés par les moyens de détection pour commander un arrêt des moyens d'alimentation lors de la présence d'un réceptacle dans ladite position angulaire.

Un tel transporteur fonctionnant sans frottement, il ne comprend aucune pièce d'usure. De plus, il présente un caractère compact et ne nécessite pas d'intervention. En outre, l'utilisation de gaz sous pression pour entraîner le plateau en rotation assure son nettoyage automatique. Enfin, l'absence de courant électrique constitue un atout majeur du point de vue de la sécurité, puisque tout risque d'explosion est supprimé.

Bien entendu, si un tel transporteur est particulièrement adapté pour être utilisé à l'intérieur d'une enceinte de confinement dans l'industrie nucléaire, on comprend aisément qu'il peut aussi être utilisé en dehors d'une telle enceinte et pour d'autres applications.

Dans un mode de réalisation particulier de l'invention, les moyens de détection à distance comprennent un détecteur optique solidaire du socle fixe et des repères formés sur le plateau tournant en face de ce détecteur, dans des plans radiaux passant par chacun des réceptacles.

Afin d'assurer le positionnement initial du plateau tournant, pour que le défilement des flacons placés dans les réceptacles s'effectue dans un ordre donné, le transporteur peut également comprendre des moyens de repérage à distance d'une position angulaire initiale du plateau. Ces moyens comprennent par exemple un deuxième détecteur optique solidaire du socle fixe et un repère formé sur le plateau tournant en face de ce deuxième détecteur, dans un plan radial passant par l'un des réceptacles.

Dans un perfectionnement particulièrement intéressant de ce transporteur, celui-ci comprend de plus des moyens d'agitation comportant un deuxième injecteur solidaire du socle fixe, des moyens d'alimentation en gaz pulsé sous pression raccordés sur ce deuxième injecteur et au moins un conduit formé dans le plateau et comprenant deux extrémités qui débouchent respectivement au fond de l'un des réceptacles et en face du deuxième injecteur, dans une position angulaire donnée du plateau.

Grâce à ces caractéristiques, on peut assurer une agitation des flacons portés par le plateau sans avoir recours à aucun mécanisme supplémentaire. On comprend aisément que ce perfectionnement est particulièrement utile lorsqu'un tel transporteur est utilisé pour effectuer un prélèvement d'échantillons, puis une dilution des échantillons prélevés. Le transporteur peut également comprendre un poste d'éjection comprenant un troisième injecteur sur lequel sont raccordés des moyens d'alimentation en gaz sous pression, et un tube ou une goulotte d'évacuation dont une extrémité est placée au-dessus de l'un des réceptacles, pour une position angulaire donnée du plateau, dans laquelle ledit conduit débouche en face du troisième injecteur.

L'invention a aussi pour objet l'application d'un tel transporteur à une installation de prélèvement d'échantillons liquides dans des flacons bouchés par des opercules.

Plus précisément, conformément à l'invention, les flacons contenant les liquides dont on désire prélever des échantillons sont placés dans les réceptacles du plateau du transporteur et l'installation est caractérisée en ce qu'elle comprend, en plus du transporteur précédemment défini, un portique latéral fixe supportant une aiguille de prélèvement verticale par l'intermédiaire de moyens pour déplacer cette aiguille verticalement entre une position haute autorisant la rotation du plateau et une position basse de prélèvement, dans laquelle l'opercule est perforé par l'aiguille, et une vanne chromatographique munie d'une boucle d'échantillonnage, cette vanne pouvant occuper soit une position de prélèvement d'échantillons dans laquelle deux premières entrées, communiquant respectivement avec l'aiguille et avec un venturi d'aspiration, sont reliées entre elles au travers de ladite boucle, soit une position d'évacuation de l'échantillon, dans laquelle deux autres entrées de la vanne, communiquant respectivement avec des moyens d'éjection d'un échantillon prélevé et avec un tube d'évacuation de l'échantillon, sont reliées entre elles au travers de ladite boucle.

Dans une telle installation, les moyens d'éjection de l'échantillon prélevé comprennent de préférence des moyens pour éjecter simultanément un volume connu de diluant liquide. Ces moyens pour éjecter un volume connu de diluant liquide peuvent notamment comprendre une deuxième vanne chromatographique dont une première entrée communiquant avec une burette est reliée à une deuxième entrée communiquant avec la première vanne, dans une première position de la deuxième vanne, cette première entrée étant reliée à une troisième entrée communiquant avec un réservoir de diluant liquide, dans une deuxième position de la deuxième vanne.

Dans ce cas, les réceptacles sont de préférence espacés d'un pas donné sur le plateau et les flacons remplis d'échantillons liquides et des flacons vides sont placés alternativement dans ces réceptacles. Le portique latéral supporte alors également une aiguille de réinjection verticale décalée dudit pas par rapport à l'aiguille de prélèvement, cette aiguille de réinjection étant supportée par l'intermédiaire de deuxièmes moyens pour déplacer cette aiguille verticalement entre une position haute autorisant la rotation du plateau et une position basse d'évacuation, dans laquelle l'opercule d'un flacon vide est perforé par l'aiguille de réinjection, cette dernière communiquant avec le tube d'évacuation de l'échantillon.

Afin que l'échantillon dilué puisse à son tour être prélevé et envoyé dans un analyseur, le portique latéral peut également supporter une aiguille verticale de prélèvement d'échantillon dilué, décalée dudit pas par rapport à l'aiguille de réinjection, cette aiguille de prélèvement d'échantillon dilué étant supportée par l'intermédiaire de troisièmes moyens pour déplacer cette aiguille verticalement entre une position haute autorisant la rotation du plateau et une position basse de prélèvement dans laquelle l'opercule d'un flacon rempli de l'échantillon dilué est perforé par l'aiguille de prélèvement de l'échantillon dilué, cette aiguille de prélèvement d'échantillon dilué communiquant avec un tube de prélèvement.

De préférence, cette installation comprend alors également une troisième vanne chromatographique munie d'une deuxième boucle d'échantillonnage, cette vanne pouvant occuper soit une position de prélèvement d'échantillon dilué, dans laquelle deux premières entrées, communiquant respectivement avec le tube de prélèvement et avec un deuxième venturi d'aspiration, sont reliées entre elles au travers de la deuxième boucle d'échantillonnage, soit une position d'évacuation de l'échantillon dilué, dans laquelle deux autres entrées de la troisième vanne, communiquant respectivement avec un moyen d'injection d'un liquide de transfert et avec un appareil d'analyse, sont reliées entre elles au travers de la deuxième boucle.

Un mode de réalisation particulier de l'invention va maintenant être décrit, à titre d'exemple non limitatif, en se référant aux dessins annexés, dans lesquels :

– la figure 1 est une vue de côté représentant schématiquement et en coupe partielle une installation de prélèvement d'échantillons liquides dans des flacons portés par un transporteur rotatif à avance pas-à-pas conforme à l'invention,

– la figure 2 est une vue en coupe du transporteur utilisé dans l'installation de la figure 1, selon un plan vertical passant par l'axe de rotation du transporteur,

– la figure 3 est une vue en perspective représentant une partie de la face inférieure du plateau tournant du transporteur de la figure 2, et

– la figure 4 représente de façon schématique l'installation de prélèvement d'échantillons de la figure 1 et, en particulier, les liaisons pneumatiques, hydrauliques et électriques entre les différents composants de cette installation.

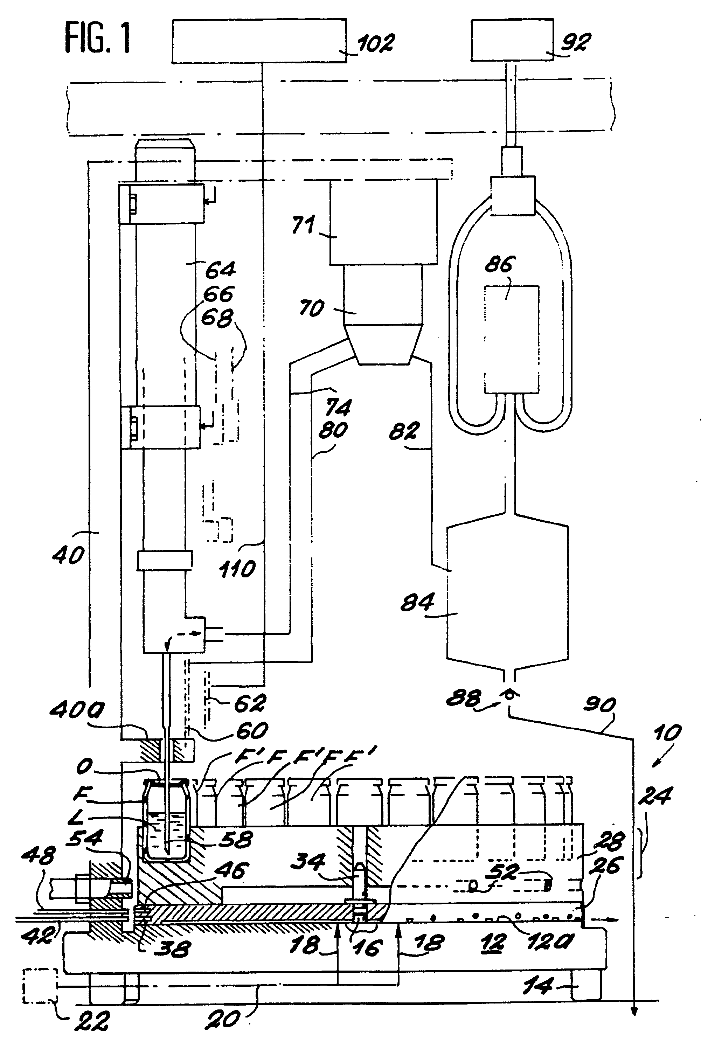

L'installation représentée de façon schématique sur la figure 1 est conçue pour effectuer de façon automatique le prélèvement d'un échantillon de volume donné dans un liquide radioactif L contenu dans un flacon F obturé par un opercule O réalisé par exemple en élastomère. Lorsque ce prélèvement est effectué, l'échantillon liquide ainsi qu'un certain volume de diluant sont réinjectés dans un flacon F' identique au flacon F mais vide. L'échantillon dilué est alors agité, puis un volume donné de cet échantillon dilué est à nouveau prélevé dans le flacon F' pour être envoyé dans un analyseur d'un type quelconque.

Avant de décrire en détail l'installation permettant de réaliser ces différentes opérations, il faut observer que l'invention n'est nullement limitée à cette application particulière. En effet, l'invention concerne en premier lieu un transporteur rotatif, quelle que soit l'application qui en est faite. De plus, si l'invention concerne également une installation de prélèvement d'échantillons liquides, ces échantillons peuvent être de nature quelconque.

Le transporteur rotatif à avance pas-à-pas désigné de façon générale par la référence 10 sur la figure 1 comprend un socle fixe 12 pouvant être posé par des pieds 14 sur une surface horizontale quelconque. Ce socle fixe 12 présente une face supérieure plane et horizontale 12a délimitée extérieurement par un bord circulaire.

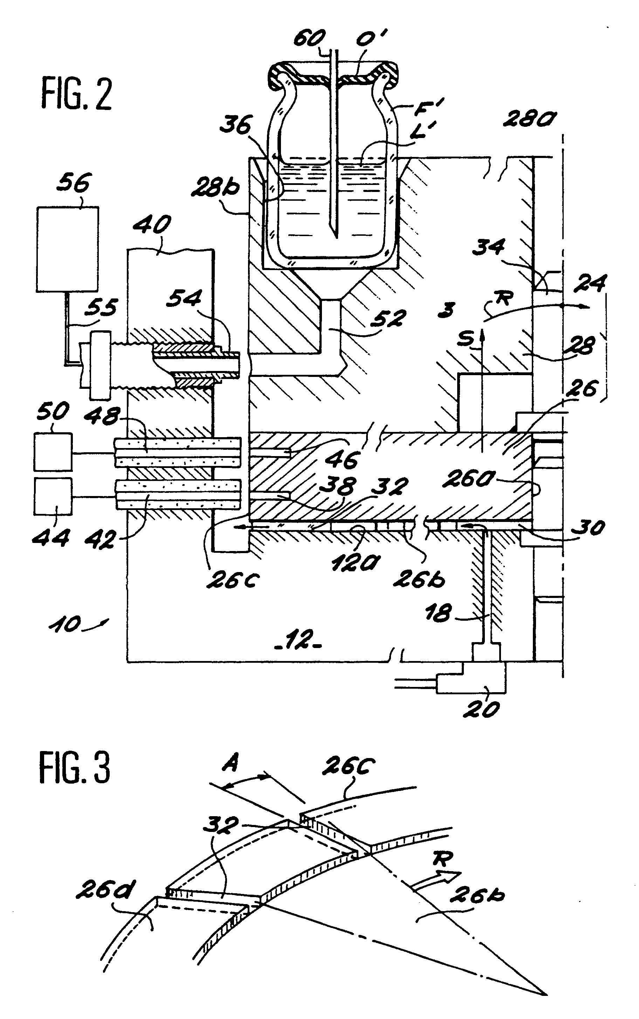

Comme le montre bien la figure 2, un plot de centrage 16 fait saillie verticalement au-delà de la face supérieure plane 12a, selon l'axe vertical de celle-ci. Des injecteurs 18, par exemple au nombre de trois, débouchent également sur la face supérieure plane 12a du socle 12, à proximité du plot de centrage 16. Ces injecteurs 18 communiquent par une tuyauterie 20 avec une source de gaz sous pression 22 (figure 1).

Le socle fixe 12 peut notamment être réalisé en plexiglas.

Un plateau tournant 24 repose par gravité sur la face supérieure 12a du socle 12. Ce plateau 24 se compose d'une platine métallique 26 sur laquelle est posée une pièce massive 28, réalisée par exemple en plexiglas.

La platine métallique 26, par exemple en acier inoxydable, est pourvue d'un alésage central 26a dans lequel vient se loger le plot de centrage 16 porté par le socle 12. Elle constitue le moteur du transporteur 10.

Cette platine 26 présente une masse élevée, par exemple d'environ 4 kg, pour un diamètre extérieur d'environ 22 cm.

La platine 26 repose sur la face supérieure plane 12a du socle 12 par une couronne périphérique 26d (figure 3) faisant saillie par rapport au reste de sa face inférieure 26b. De cette manière, les injecteurs 18 débouchent dans une chambre centrale 30 (figure 2) délimitée entre la face supérieure plane 12a du socle et la face inférieure 26b de la platine.

Comme l'illustre bien la figure 3, des rainures 32 sont formées dans la couronne 26d, pour faire communiquer la chambre centrale 30 avec le bord extérieur cylindrique 26c de la platine 26. Ces rainures 32 sont régulièrement réparties sur toute la périphérie de la couronne et elles sont toutes inclinées dans le même sens et d'un même angle A par rapport aux rayons passant par l'axe vertical du plateau 24.

Grâce aux caractéristiques qui viennent d'être décrites, l'arrivée d'un gaz sous pression par les injecteurs 18 a deux effets distincts sur le plateau 24.

Un premier de ces effets est la sustentation du plateau résultant de l'arrivée du gaz sous pression dans la chambre centrale 30 (flèche S sur la figure 2). Le second effet est une mise en rotation lente du plateau 24 résultant de l'échappement du gaz comprimé admis dans la chambre centrale 30 par les rainures 32. En effet, l'inclinaison de ces rainures par rapport aux rayons passant par l'axe de rotation du plateau crée une composante tangentielle faisant tourner lentement le plateau dans le sens de la flèche R sur les figures 2 et 3.

On comprendra aisément que des effets analogues pourraient être obtenus en donnant aux rainures 32 des formes différentes, par exemple incurvées, ou en remplaçant ces rainures par des passages traversant la platine 26 de façon à mettre en communication la chambre centrale 30 avec le bord extérieur cylindrique 26c de la platine.

Conformément à une caractéristique importante de l'invention, la masse de la platine 26 est suffisamment élevée pour que la vitesse d'entraînement en rotation du plateau 24 reste faible et pour que la rotation du plateau soit stoppée pratiquement instantanément lorsque l'arrivée de gaz sous pression par les injecteurs 18 est interrompue.

La platine 26 porte au centre de sa face supérieure plane un plot de centrage 34 qui vient se loger dans un alésage 28a formé au centre de la pièce 28 du plateau tournant 24. Cette pièce 28 repose simplement par gravité sur la platine 26 de façon à tourner avec celle-ci. Cette pièce 28 sert à supporter les flacons F dans lesquels doivent être prélevés les échantillons liquides, ainsi que les flacons F' vides dans lesquels sont réinjectés les échantillons liquides.

La pièce 28 présente un bord extérieur cylindrique 28b de même diamètre que le bord extérieur cylindrique 26c de la platine 26. Des évidements 36 constituant des réceptacles pour les flacons F et F' sont formés dans la partie extérieure de la face supérieure de la pièce 28. Ces évidements sont régulièrement répartis afin de définir entre chacun d'entre eux un pas angulaire déterminé autour de l'axe vertical de rotation du plateau 24. Ces évidements 36 ont des formes identiques adaptées aux formes des flacons F et F' qu'ils doivent recevoir. De plus, ils présentent des axes verticaux qui sont disposés à égale distance de l'axe de rotation du plateau. Dans l'exemple représenté, les évidements 36 sont de forme cylindrique.

A titre d'exemple nullement limitatif, le plateau 24 peut être pourvu de vingt-quatre réceptacles 36 séparés les uns des autres d'un pas angulaire de 15°.

Il est à noter que ce transporteur est adaptable à d'autres formes de flacons ou à des récipients de types différents, en remplaçant la pièce massive 28 par une pièce présentant des évidements de formes différentes.

Afin de repérer l'arrivée de chacun des réceptacles 36 dans une position angulaire donnée lors de la rotation du plateau tournant 24, des trous borgnes 38 orientés radialement sont percés dans le bord extérieur 26c de la platine 26, dans un même plan horizontal, ces trous 38 étant répartis angulairement selon le même pas que celui qui sépare les réceptacles 36. Grâce à un doigt de positionnement angulaire (non représenté) formé sur la platine 26 et venant se loger dans une encoche formée sur la face inférieure de la pièce 28, celle-ci est positionnée automatiquement de telle sorte que chacun des trous borgnes 38 soit situé dans un plan radial donné, passant par exemple par l'axe vertical de l'un des réceptacles 36.

Comme l'illustrent les figures 1 et 2, un portique latéral 40 solidaire du socle 12 du transporteur 10, supporte différents organes de l'installation de prélèvement d'échantillons qui sera décrite ultérieurement. Afin de détecter l'arrivée de l'un des réceptacles 36 du plateau tournant en face de l'un des organes portés par le portique 40, ce dernier supporte l'extrémité d'une fibre optique 42, orientée radialement par rapport à l'axe de rotation vertical du plateau 24 et située dans le même plan horizontal que les trous borgnes 38. En plaçant à l'autre extrémité de la fibre optique 42 un dispositif émetteur-récepteur 44 (figure 2), on détecte instantanément l'arrivée d'un trou 38 en face de l'extrémité de la fibre optique 42 par la disparition du signal lumineux renvoyé normalement par la surface extérieure réfléchissante 26c de la platine 26.

On comprend que la détection de l'arrivée d'un trou borgne 38 en regard de la fibre optique 42 permet de commander automatiquement l'arrêt de l'injection de gaz sous pression par les injecteurs 18. Compte tenu des caractéristiques décrites précédemment, le plateau tournant 24 s'arrête alors instantanément dans la position angulaire souhaitée et les opérations correspondantes peuvent être accomplies.

Afin que ces opérations soient effectuées dans un ordre donné à partir d'un flacon F déterminé à l'avance, on voit sur les figures 1 et 2 qu'un trou borgne unique 46 orienté radialement comme les trous borgnes 38 et disposés dans le même plan radial que l'un d'entre eux, mais dans un plan horizontal différent, peut être formé sur le bord extérieur 26c de la platine 26, au droit du réceptacle 36 contenant le flacon F dans lequel on désire prélever le premier échantillon.

L'extrémité d'une deuxième fibre optique 48 est alors fixée sur le portique 40, de façon à être orientée radialement et située dans le même plan horizontal que le trou borgne 46. Comme la fibre optique 42, la fibre 48 est reliée par son autre extrémité à un dispositif émetteur-récepteur optique 50 émettant un signal d'initialisation lorsque le trou borgne 46 se trouve en face de l'extrémité de la fibre optique 48.

Lorsqu'un tel dispositif d'initialisation du procédé est prévu, les signaux émis par le détecteur 44 ne sont pas transmis à la source de gaz sous pression 22 tant qu'un signal d'initialisation n'a pas été émis par le détecteur 50. Le plateau 24 tourne donc jusqu'à ce que le trou borgne 46 arrive en regard de l'extrémité de la fibre optique 48. L'avance pas-à-pas du plateau tournant s'effectue ensuite de la manière décrite précédemment.

Bien entendu, les ensembles constitués par les trous 38 et la fibre optique 42, ainsi que par le trou 46 et la fibre optique 48 peuvent être remplacés par tout autre dispositif de repérage sans contact des positions angulaires du plateau 24. En outre, les orientations des trous et des extrémités correspondantes des fibres peuvent être différentes de celles qui ont été décrites.

Dans l'exemple de réalisation représenté sur les figures, le transporteur 10 comprend également des moyens pour agiter les flacons F′ lorsque l'échantillon de liquide dilué L′ (figure 2) a été introduit dans ces flacons.

Comme on le verra ultérieurement, les réceptacles 36 reçoivent alternativement un flacon F contenant un liquide dont on désire prélever un échantillon et un flacon F′ initialement vide. Les moyens pour agiter les flacons concernent uniquement les réceptacles contenant les flacons F′, seul un réceptacle sur deux est équipé de ces moyens.

Comme l'illustre la figure 2, ces moyens d'agitation comprennent un passage 52 reliant le fond de chaque réceptacle destiné à recevoir un flacon F′ avec le bord extérieur cylindrique 28b de la pièce 28. Plus précisément, l'extrémité du passage 52 qui débouche sur la face extérieure 28b est orientée radialement par rapport à cette face alors que l'extrémité du passage 52 qui débouche dans le fond du réceptacle 36 est orientée selon l'axe vertical de ce réceptacle.

Pour permettre l'agitation, on voit sur la figure 2 que le portique 40 supporte également un injecteur 54 orienté radialement et disposé dans un plan horizontal contenant les extrémités des passages 52 qui débouchent sur la face extérieure 28b de la pièce 28.

L'injecteur 54 communique par un tube 55 avec une source de gaz pulsé sous pression 56 (générateur de fréquences pneumatiques). De cette manière, lorsque l'injecteur 54 est en face de l'extrémité de l'un des passages 52 et lorsque la source 56 est actionnée, le flacon F′ placé dans le réceptacle 36 correspondant est animé d'un mouvement de va-et-vient selon une direction verticale sous l'effet de l'air pulsé injecté par le passage 52.

De préférence, et comme l'illustre la figure 1, le portique 40 est équipé d'une plaque horizontale 40a située alors verticalement au-dessus du flacon F′ à une distance telle que celui-ci ne peut s'échapper de son réceptacle 36 lors de son agitation.

Une partie des moyens d'agitation qui viennent d'être décrits peut aussi être utilisée pour éjecter les flacons F′, lorsque toutes les opérations sont terminées. A cet effet, on prévoit un poste d'éjection comportant un autre injecteur comparable à l'injecteur 54 et sur lequel est raccordée une source de gaz sous pression. L'arrivée du gaz sous pression par le passage 52 débouchant sous le flacon permet d'éjecter ce dernier, par exemple dans une goulotte ou dans un tube d'évacuation placé au-dessus du flacon.

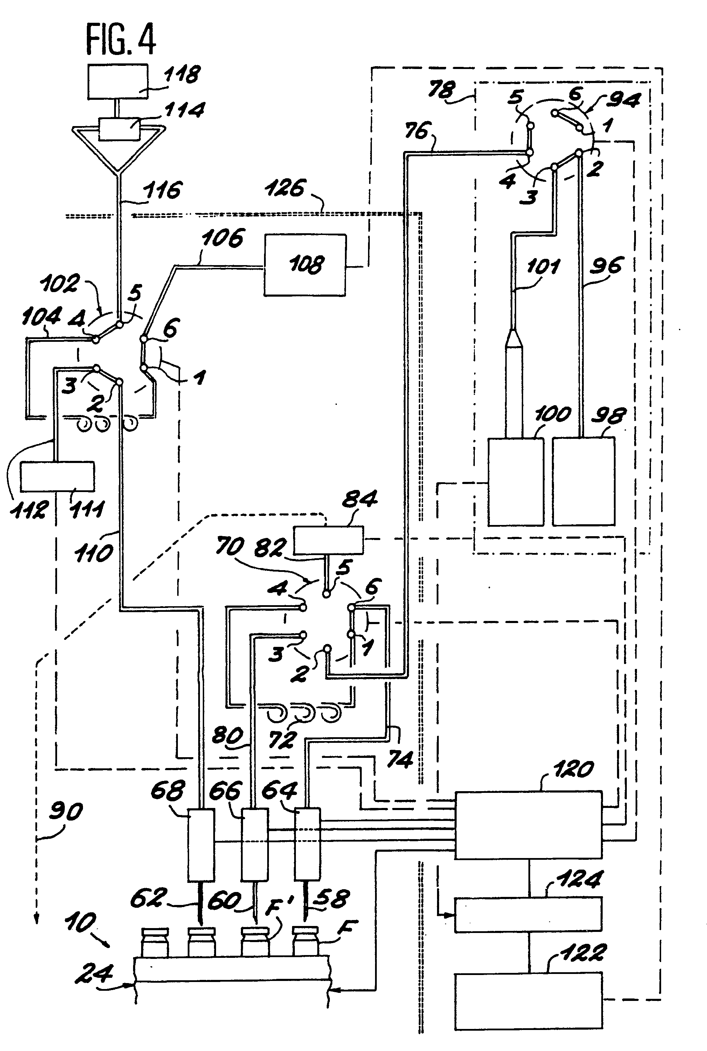

La description de l'installation de prélèvement d'échantillons liquides incorporant le transporteur rotatif à avance pas-à-pas 10 va maintenant être faite en se référant plus précisément aux figures 1 et 4.

Comme on le voit sur la figure 1 et, de façon plus schématique, sur la figure 4, le portique 40 supporte trois aiguilles verticales désignées par les références 58, 60 et 62. Ces aiguilles sont disposées de telle sorte qu'elles se trouvent toutes les trois au-dessus de trois flacons successifs portés par le plateau 24 lorsqu'un trou borgne 38 est en face de la fibre optique 42.

Plus précisément, l'aiguille 58 constitue une aiguille de prélèvement d'échantillons, l'aiguille 60 étant une aiguille de réinjection de l'échantillon dilué et l'aiguille 62 une aiguille de prélèvement d'échantillon dilué. Le sens de rotation du plateau tournant 24 est tel que chacun des flacons F et F′ défile successivement devant les aiguilles 58, 60 et 62.

Chacune des aiguilles 58, 60 et 62 est supportée par le portique 40 par l'intermédiaire d'un vérin à double effet 64, 66 et 68 respectivement. Chacun de ces vérins permet de déplacer verticalement l'aiguille correspondante entre une position haute dans laquelle l'aiguille est totalement dégagée du flacon et permet la rotation du plateau 24 et une position basse dans laquelle l'aiguille correspondante traverse l'opercule O ou O′ obturant le flacon F ou F′ pour plonger dans ce dernier.

Le portique 40 supporte également une vanne chromatographique 70 dont la configuration apparaît plus précisément sur la figure 4. Cette vanne 70 est pourvue de six entrées numérotées de 1 à 6 sur la figure 4. Les entrées 1 et 4 sont reliées entre elles par un boucle d'échantillonnage 72 dont la longueur détermine le volume de l'échantillon à prélever. L'entrée n°6 de la vanne 70 communique par un tube 74 avec l'aiguille de prélèvement d'échantillon 58. L'entrée n°2 communique par un tube 76 avec un dispositif d'injection de diluant liquide désigné de façon générale par la référence 78 et dont la description plus détaillée sera faite ultérieurement. L'entrée n°3 de la vanne chromatographique 70 communique directement avec l'aiguille 60 de réinjection de l'échantillon dilué par un tube 80. Enfin, l'entrée n°5 de la vanne 70 communique par un tube 82 avec une ampoule à vide 84, également supportée par le portique latéral 40, comme le montre la figure 1.

La vanne 70 est équipée d'un actuateur pneumatique 71 (figure 1) commandé à distance comme on le verra ultérieurement.

Tous les tubes 72, 74, 76, 80 et 82 sont réalisés en acier inoxydable.

Comme l'illustre la figure 1, l'ampoule à vide 84 communique par son extrémité supérieure avec un venturi 86. L'ampoule à vide 84 est pourvue à son extrémité inférieure d'un clapet anti-retour 88 permettant d'évacuer les reliquats du liquide prélevé vers un système d'égout par une tuyauterie 90. Le venturi 86 est commandé à distance par un compresseur 92.

Dans une première position, dite de prélèvement d'échantillon, de la vanne 70 représentée sur la figure 4, les entrées 1 et 6 sont reliées entre elles, de même que les entrées 2 et 3 et les entrées 4 et 5. L'actionnement de la vanne 70 permet de la faire passer dans une deuxième position dans laquelle les entrées 1 et 2, 3 et 4, 5 et 6 sont reliées entre elles. Cette deuxième position est appelée position d'évacuation de l'échantillon.

Lors du fonctionnement du transporteur 10, on a vu que la rotation du plateau 24 commandée par l'injection d'air comprimé au travers des injecteurs est interrompue automatiquement lorsqu'un trou 38 arrive en face de la fibre optique 42. Le plateau est alors immobilisé de telle sorte que l'aiguille 58 se trouve au-dessus d'un flacon F contenant le liquide à analyser et que l'aiguille 60 se trouve au-dessus d'un flacon F' vide.

Le vérin 64 est ensuite actionné automatiquement afin d'introduire l'aiguille 58 dans le flacon F correspondant. La vanne 70 étant dans la position de prélèvement d'échantillons représentée sur la figure 4, l'aiguille 58 de prélèvement d'échantillons est reliée au venturi 86 par l'intermédiaire de la boucle d'échantillonnage 72.

Un actionnement du compresseur 92 relié au venturi 84 a alors pour effet d'aspirer une partie du liquide contenu dans le flacon F jusque dans l'ampoule à vide 84, dont le fond est alors obturé par le clapet anti-retour 88. La boucle d'échantillonnage 72 est ainsi remplie. On commute alors la vanne 70 pour l'amener dans sa position d'évacuation de l'échantillon. Le compresseur 92 est ensuite stoppé, ce qui a pour effet de faire disparaître le vide dans l'ampoule 86. Le reliquat de liquide présent dans cette ampoule est alors évacué automatiquement par gravité vers l'égout en raison de l'ouverture automatique du clapet anti-retour 88.

Lors de la rotation de la vanne 70, une quantité bien déterminée de liquide est emprisonnée dans la boucle d'échantillonnage 72. Après la rotation de la vanne, cet échantillon de liquide prélevé se trouve placé entre le dispositif 78 d'injection de liquide de dilution et l'aiguille 60 de réinjection de l'échantillon dilué.

Après avoir perforé l'opercule O' du flacon F' en actionnant le vérin 66 commandant l'aiguille 60, on évacue ensuite l'échantillon liquide emprisonné dans la boucle 72, ainsi qu'une certaine quantité de liquide de dilution, à l'intérieur du flacon F', initialement vide, grâce au dispositif 78.

Dans l'exemple de réalisation représenté sur la figure 4, ce dispositif 78 d'injection de liquide de dilution comprend une autre vanne chromatographique 94 dont les entrées sont également numérotées de 1 à 6. Le tube 76 est raccordé sur l'entrée n°4 de cette vanne 94. Par ailleurs l'entrée n°2 de cette vanne communique par un tube 96 avec un réservoir 98 contenant le liquide de dilution. L'entrée n°3 de la vanne 94 communique quant à elle avec une burette de dosage 100 par un tube 101. Enfin, les entrées 1, 5 et 6 sont obturées par des bouchons.

La rotation de la vanne 94 est assurée par un actuateur pneumatique (non représenté).

Dans la position de la vanne 94 représentée sur la figure 4, les entrées 2 et 3 sont reliées entre elles, de sorte qu'un volume donné de diluant peut être aspiré par la burette 100 dans le réservoir 98. A cet effet, la burette 100 est équipée d'une motorisation pas-à-pas. Cette opération d'aspiration est effectuée avant que la vanne 70 ne passe dans sa position d'évacuation de l'échantillon. La vanne 94 est ensuite commutée dans une deuxième position dans laquelle les entrées 3 et 4 sont reliées entre elles. C'est dans cette position que le refoulement simultané de la quantité de liquide de dilution contenue dans la burette 100 ainsi que l'échantillon de liquide prélevé contenu dans la boucle 72 peuvent être refoulés dans le flacon F' par l'aiguille 60. Ce refoulement est commandé par la burette 100.

On comprendra aisément que le dispositif 78 pourrait être réalisé différemment. En particulier, la vanne chromatographique 94 peut être remplacée par une simple vanne à trois voies à fonctionnement pneumatique. L'ensemble formé par la burette 100 et par la vanne peut aussi être remplacé par tout système équivalent tel qu'un piston doseur.

Lorsque l'échantillon dilué a été évacué par l'aiguille 60 dans le flacon F' de la manière qui vient d'être décrite, les aiguilles 58 et 60 sont remontées. Une agitation de l'échantillon est alors généralement nécessaire, afin que la concentration de celui-ci à l'intérieur du diluant soit aussi homogène que possible. C'est donc à ce moment que les moyens d'agitation du flacon F' décrits précédemment en se référant à la figure 2 sont mis en oeuvre. A cet effet, l'injecteur 54 est situé dans le plan radial passant par l'axe du plateau 24 et contenant l'aiguille 60.

Lorsque l'agitation est terminée, l'échantillon dilué contenu dans le flacon F', ou au moins une partie de cet échantillon, doit être prélevé et évacué vers un appareil d'analyse d'un type quelconque adapté aux mesures à effectuer (par exemple, mesures spectrophotométriques ou spectrofluorimétriques, mesures de radioactivité, de pH, de résistivité, de turbidimétrie, etc.).

A cet effet, une rotation du plateau tournant 24 est alors commandée afin d'amener le flacon F' dans lequel vient d'être introduit l'échantillon dilué en-dessous de l'aiguille 62. Le vérin 68 est ensuite actionné afin d'introduire l'extrémité de l'aiguille 62 dans la solution contenue dans le flacon F'.

Afin d'effectuer le prélèvement de l'échantillon dilué contenu dans le flacon F' lorsque ce dernier arrive en-dessous de l'aiguille 62, l'installation comprend de plus, dans l'exemple de réalisation représenté sur la figure 4, une autre vanne chromatographique 102 qui peut également être montée sur le portique 40. Les six entrées de cette vanne 102 sont numérotées de 1 à 6 sur la figure 4.

Dans l'exemple représenté, la vanne chromatographique 102 est également équipée d'une boucle d'échantillonnage 104 reliant ces entrées 1 et 4 et dont la longueur permet de déterminer le volume d'échantillon dilué envoyé vers un appareil d'analyse 108. L'entrée n°6 de la vanne 102 est reliée par un tube 106 à cet appareil d'analyse 108. L'entrée n°2 est reliée quant à elle à l'aiguille de prélèvement d'échantillon dilué 62 par un tube 110. Par ailleurs, une chambre à vide 111 communique avec l'entrée n°3 de la vanne 102 par un tube 112. Cette chambre à vide 111 est analogue à la chambre 84 et elle communique de la même manière avec un venturi et avec un système d'égout (non représentés). Enfin, l'entrée n°5 de la vanne 102 est reliée à une pompe 114 par un tube 116.

La vanne 102 est représentée sur la figure 4 dans sa position d'évacuation de l'échantillon prélevé dans la boucle 104 vers l'analyseur 108. Dans cette position, les entrées 1 et 6, 2 et 3 et 4 et 5 de la vanne communiquent entre elles.

Dans une position de prélèvement de l'échantillon dilué, ce sont au contraire les entrées 1 et 2, 3 et 4 et 5 et 6 qui sont reliées entre elles. Dans cette position, l'aiguille 62 est reliée à la chambre à vide 111 par l'intermédiaire de la boucle d'échantillonnage 104. Le prélèvement de l'échantillon dilué dans le flacon F' s'effectue donc de la même manière que le prélèvement de l'échantillon non dilué contrôlé par la vanne chromatographique 70. Ainsi, l'actionnement du venturi associé à la chambre à vide 111 a pour effet de remplir la boucle d'échantillonnage 104 d'échantillon dilué.

Pour refouler l'échantillon dilué piégé dans la boucle 104 vers l'analyseur 108, la vanne 102 est amenée dans la position représentée sur la figure 4. Dans ces conditions, la boucle d'échantillonnage 104 se trouve située entre la pompe 114 et l'analyseur 108.

L'actionnement de la pompe 114 a alors pour effet de refouler l'échantillon dilué contenu dans la boucle 104 vers l'analyseur 108 au moyen d'un liquide de transfert prélevé dans un réservoir 118.

L'exemple de réalisation représenté s'applique au cas où l'analyse de l'échantillon dilué nécessite le prélèvement d'un volume précis de solution. En variante, si l'analyse ne nécessite aucune précision en volume, la solution peut être aspirée directement dans l'analyseur. Dans ce cas, l'analyseur 108 est placé entre l'aiguille de prélèvement d'échantillon dilué 62 et l'ampoule à vide 111. La vanne 102 ainsi que la pompe 114 et son réservoir associés 118 sont alors supprimés. Dans ce cas, une mesure en statique peut être effectuée si nécessaire en arrêtant le venturi avant aspiration complète de la solution.

Comme on l'a représenté schématiquement sur la figure 4, l'installation comprend de plus un boîtier d'électrovannes 120 servant à commander l'ensemble des organes pneumatiques de l'installation. Ce boîtier 120 commande les vérins 64, 66 et 68 associés à chacune des aiguilles ainsi que la mise en communication des sources de gaz comprimé 22 et 56 respectivement avec les injecteurs 18 et 54. Le boîtier 120 commande de plus les actuateurs pneumatiques des trois vannes chromatographiques 70, 94 et 102 ainsi que la mise en oeuvre des venturis 84 et 111.

Le boîtier d'électrovannes 120 est commandé par une logique de commande programmable 122, au travers d'une interface 124. La logique de commande 122 est sensible aux signaux délivrés par les détecteurs optiques 44 et 50, afin de commander le boîtier d'électrovannes 120 et le moteur pas-à-pas de la burette 100 au travers de l'interface 124, selon un programme déterminé. La logique 122 peut aussi être utilisée pour traiter les informations fournies par l'analyseur 108.

Sur la figure 4, on a également représenté en 126 une partie de la paroi de la cellule de confinement à l'intérieur de laquelle s'effectue le prélèvement, dans le cas de l'industrie nucléaire. Cette illustration fait apparaître clairement qu'en dehors du transporteur rotatif 10 et des aiguilles 58, 60 et 62, des vannes 70 et 102 et des venturis 84 et 110, qui sont montés sur le portique latéral 40, et du doseur 108, tous les autres organes de l'installation sont placés à l'extérieur de la cellule. Cette configuration permet de limiter l'encombrement et le coût des parties de l'installation situées à l'intérieur de la cellule et simplifie la maintenance. De plus, tous les organes présents à l'intérieur de la cellule sont à actionnement par air comprimé, ce qui permet d'éviter tout risque d'explosion et d'améliorer la sécurité.

De plus, la qualité et la reproductibilité des prélèvements se trouvent améliorées par l'utilisation de vannes chromatographiques à boucles d'échantillonnage dont la précision est bien connue. L'ensemble géré par la logique de commande 122 est en outre entièrement automatisé une fois que les flacons F et F' ont été logés dans leurs réceptacles. La mise en place des flacons sur le plateau ne fait pas partie de l'invention. Cependant, on comprendra aisément qu'elle peut également être automatisée, si nécessaire.

Par ailleurs, comme on la déjà mentionné à plusieurs reprises, l'installation de prélèvement d'échantillons liquides selon l'invention peut subir différentes modifications sans sortir du cadre de l'invention, telle que définie dans les revendications. L'installation peut notamment être très simplifiée si la dilution de l'échantillon prélevé n'est pas nécessaire. Dans ce cas, le portique 40 supporte une seule aiguille de prélèvement 58 et la vanne 70 équipée de son venturi 84. Le dispositif 78 est alors remplacé par un ensemble constitué par une pompe et par un réservoir de liquide de transfert comparables à la pompe 114 et au réservoir 118. Dans ce cas, la sortie 3 de la vanne 70 est raccordée directement sur l'analyseur 108.

Il est aussi possible de disposer en série plusieurs vannes chromatographiques de prélèvement analogues à la vanne 70, chacune de ces vannes étant équipée d'une boucle d'échantillonnage de longueur différente. Plusieurs volumes différents peuvent alors être prélevés, d'un flacon à l'autre, selon la nature des analyses à effectuer.

Enfin, la ou les vannes chromatographiques peuvent ne pas être supportées par le portique.

## Revendications

1. Transporteur rotatif à avance pas-à-pas, comprenant :
   – un socle fixe (12) pourvu d'une face supérieure horizontale (12a) sur laquelle débouche au moins un injecteur (18) communiquant avec des moyens d'alimentation en gaz sous pression (22),
   – un plateau tournant (24) reposant par gravité sur la face supérieure du socle et coopérant avec cette face par des moyens de centrage (16, 26a) du plateau autour d'un axe vertical fixe, ce plateau ayant un bord extérieur circulaire (26c, 28b), une face supérieure pourvue de réceptacles (36) répartis en couronne autour dudit axe et une face inférieure (26b) délimitant avec la face supérieure (12a) du socle une chambre centrale de sustentation (30), au moins un passage (32) orienté selon une direction inclinée par rapport à un rayon passant par ledit axe étant formé dans le plateau (24) pour relier la chambre centrale de sustentation et le bord extérieur du plateau, afin d'assurer une rotation de ce dernier lorsque les moyens d'alimentation (22) sont actionnés, le plateau ayant un poids élevé tel que cette rotation soit stoppée instantanément lorsque les moyens d'alimentation sont arrêtés,
   – des moyens de détection à distance (38, 42, 44) de la présence d'un réceptacle (36) dans une position angulaire donnée autour dudit axe,
   – des moyens de commande (122, 120) sensibles à des signaux délivrés par les moyens de détection (44) pour commander un arrêt des moyens d'alimentation (22) lors de la présence d'un réceptacle (36) dans ladite position angulaire.

2. Transporteur selon la revendication 1, caractérisé en ce que les moyens de détection à distance comprennent un détecteur optique (42) solidaire du socle fixe et des repères (38) formés sur le plateau tournant (24) en face de ce détecteur, dans des plans radiaux associés à chacun des réceptacles.

3. Transporteur selon l'une quelconque des revendications 1 et 2, caractérisé en ce qu'il comprend de plus des moyens de repérage à distance d'une position angulaire initiale du plateau, comprenant un deuxième détecteur optique (48) solidaire du socle fixe et un repère (46) formé sur le plateau tournant (24) en face de ce deuxième détecteur, dans un plan radial passant par l'un des réceptacles (36).

4. Transporteur selon l'un quelconque des revendications 1 à 3, caractérisé en ce qu'il comprend de plus des moyens d'agitation, comprenant un deuxième injecteur (54) solidaire du socle fixe, des moyens d'alimentation en gaz pulsé sous pression (56) raccordés sur ce deuxième injecteur et au moins un conduit (52) formé dans le plateau (24) et comprenant deux extrémités qui débouchent respectivement au fond de l'un des réceptacles (36) et en face du deuxième injecteur, dans une position angulaire donnée du plateau.

5. Transporteur selon la revendication 4, caractérisé en ce qu'il comprend de plus au moins un poste d'éjection comprenant un troisième injecteur sur lequel sont raccordés des moyens d'alimentation en gaz sous pression, et un tube ou une goulotte d'évacuation dont une extrémité est placée au-dessus de l'un des réceptacles (36), pour une position angulaire donnée du plateau, dans laquelle ledit conduit (52) débouche en face du troisième injecteur.

6. Installation de prélèvement d'échantillons liquides dans des flacons (F) bouchés par des opercules (O), caractérisée en ce qu'elle comprend un transporteur (10) selon l'une quelconque des revendications 1 à 5, les flacons étant placés dans les réceptacles (36) du plateau, cette installation comprenant de plus un portique latéral fixe (40) supportant une aiguille de prélèvement verticale (58) par l'intermédiaire de moyens (64) pour déplacer cette aiguille verticalement entre une position haute autorisant la rotation du plateau et une position basse de prélèvement dans laquelle l'opercule est perforé par l'aiguille, et une vanne chromatographique (70) munie d'une boucle d'échantillonnage (72), cette vanne pouvant occuper une position de prélèvement d'échantillons dans laquelle deux premières entrées (6, 5) communiquant respectivement avec l'aiguille (58) et avec un venturi d'aspiration (86) sont reliées entre elles au travers de ladite boucle (72), et une position d'évacuation de l'échantillon, dans laquelle deux autres entrées (2, 3) de la vanne, communiquant respectivement avec des moyens d'éjection (78) d'un échantillon prélevé et avec un tube (80) d'évacuation de cet échantillon, sont reliées entre elles au travers de ladite boucle (72).

7. Installation selon la revendication 6, caractérisée en ce que les moyens d'éjection de l'échantillon prélevé comprennent des moyens (78) pour éjecter simultanément un volume connu de diluant liquide.

8. Installation selon la revendication 7, caractérisée en ce que les moyens pour éjecter un volume connu de diluant liquide comprennent une deuxième vanne chromatographique (94) dont une première entrée (3) communiquant avec une burette (100) est reliée à une deuxième entrée (4) communiquant avec la première vanne (70), dans une première position de la deuxième vanne, cette première entrée (3) étant reliée à une troisième entrée (2) communiquant avec un réservoir de diluant liquide (98), dans une deuxième position de la deuxième vanne.

9. Installation selon l'une quelconque des revendications 6 à 8, caractérisée en ce que, les réceptacles (36) étant espacés d'un pas donné sur le plateau (24) et des flacons (F) remplis d'échantillons liquides et des flacons vides (F') étant placés alternativement dans les réceptacles, le portique latéral (40) supporte également une aiguille de réinjection verticale (60) décalée dudit pas par rapport à l'aiguille de prélèvement (58), cette aiguille de réinjection étant supportée par l'intermédiaire de deuxièmes moyens (66) pour déplacer cette aiguille verticalement entre une position haute autorisant la rotation du plateau et une position basse d'évacuation, das laquelle l'opercule (O') d'un flacon vide (F') est perforé par l'aiguille de réinjection (60), cette dernière communiquant avec le tube d'évacuation de l'échantillon (80).

10. Installation selon la revendication 9, caractérisée en ce que le portique latéral (40) supporte également une aiguille verticale de prélèvement d'échantillon dilué (62), décalée dudit pas par rapport à l'aiguille de réinjection (60), cette aiguille de prélèvement d'échantillon dilué étant supportée par l'intermédiaire de troisièmes moyens (68) pour déplacer cette aiguille verticalement entre une position haute autorisant la rotation du plateau et une position basse de prélèvement, dans laquelle l'opercule (O') d'un flacon (F') rempli de l'échantillon dilué est perforé par l'aiguille de prélèvement de l'échantillon dilué (62), cette aiguille de prélèvement d'échantillon dilué communiquant avec un tube de prélèvement (110).

11. Installation selon la revendication 10, caractérisée en ce qu'elle comprend une troisième vanne chromatographique (102) munie d'une deuxième boucle d'échantillonnage (104), cette vanne pouvant occuper soit une position de prélèvement d'échantillon dilué, dans laquelle deux premières entrées (2, 3), communiquant respectivement avec le tube de prélèvement (110) et avec un deuxième venturi d'aspiration, sont reliées entre elles au travers de la deuxième boucle d'échantillonnage (104), soit une position d'évacuation de l'échantillon dilué, dans laquelle deux autres entrées (5, 6) de la troisième vanne, communiquant respectivement avec un moyen d'injection (114) d'un liquide de transfert et avec un appareil d'analyse (108), sont reliées entre elles au travers de la deuxième boucle (104).

**Patentansprüche**

1. Schrittweise fortschreitende, rotierende Transportvorrichtung mit :
– einem festen Sockel (12), der mit einer horizontalen oberen Seite (12a) versehen ist, auf der wenigstens ein Injektionsrohr (18) mündet, das mit Versorgungsvorrichtungen für unter Druck stehendes Gas (22) in Verbindung steht,
– einer sich drehenden Platte (24), die durch Schwerkraft auf der oberen Seite des Sockels ruht und mit dieser Seite über Zentriervorrichtungen (16, 26a) der Platte um eine feste vertikale Achse zusammenwirkt, wobei diese Platte einen kreisförmigen, äußeren Rand (26c, 28b), eine obere Seite, die mit Behältern (36) versehen ist, die in einer Krone um die Achse verteilt sind, und eine untere Seite (26b) aufweist, die mit der oberen Seite (12a) des Sockels eine zentrale Auftriebskammer (30) bildet, wobei wenigstens ein Durchgang (32), der entlang einer bezüglich eines durch die Achse gehenden Strahls geneigten Richtung in die Platte (24) gebildet ist, um die zentrale Auftriebskammer und den äußeren Rand der Platte miteinander zu verbinden, um eine Rotation der letzteren sicherzustellen, wenn die Versorgungsvorrichtungen (22) betätigt werden, wobei die Platte ein erhöhtes Gewicht besitzt, damit diese Rotation sofort gestoppt wird, sobald die Versorgungsvorrichtungen angehalten werden,
– Ferndetektionsvorrichtungen (38, 42, 44) für die Anwesenheit eines Behälters (36) in einer gegebenen Winkelposition um diese Achse,
– Steuerungsvorrichtungen (122, 120), die auf von den Detektionsvorrichtungen (44) erzeugte Signal reagieren, um ein Anhalten der Versorgungsvorrichtungen (22) bei der Anwesenheit eines Behälters (36) in der besagten Winkelposition zu bewirken.

2. Transportvorrichtung nach Anspruch 1, dadurch gekennzeichnet, daß die Ferndetektionsvorrichtungen einen optischen Detektor (42), der am festen Sockel befestigt ist, und Markierungen (38) umfassen, die auf der rotierenden Platte (24) gegenüber dem Detektor in radialen, mit jedem der Behälter verbundenen Ebenen gebildet sind.

3. Transportvorrichtung nach einem der Ansprüche 1 und 2, dadurch gekennzeichnet, daß sie außerdem Vorrichtungen zum Fernorten einer anfänglichen Winkelposition der Platte umfaßt, die einen zweiten optischen Detektor (48), der am festen Sockel befestigt ist, und eine Markierung (46) umfassen, die auf der rotierenden Platte (24) gegenüber dem zweiten Detektor in einer radialen Ebene, die durch einen der Behälter (36) geht, gebildet ist.

4. Transportvorrichtung nach einem der Ansprüche 1 bis 3, dadurch gekennzeichnet, daß sie außerdem Rührvorrichtungen, die ein zweites an dem festen Sockel befestigtes Injektionsrohr umfassen, Vorrichtungen zum Versorgen mit gepulstem, unter Druck stehendem Gas (56), die auf dem zweiten Injektionsrohr befestigt sind, und wenigstens ein Rohr (52) umfaßt, das in der Platte (24) gebildet ist und zwei Enden aufweist, die jeweils am Boden von einem der Behälter (36) und gegenüber dem zweiten Injektionsrohr in einer gegebenen Winkelposition der Platte enden.

5. Transportvorrichtung nach Anspruch 4, dadurch gekennzeichnet, daß sie außerdem wenigstens einen Ausstoßposten, der ein drittes Injektionsrohr umfaßt, auf dem Vorrichtungen zur Versorgung mit unter Druck stehendem Gas befestigt sind, und ein Absaugrohr oder eine Absaugrinne umfaßt, von der ein Ende über einem der Behälter (36) für eine gegebene Winkelposition der Platte angeordnet ist und in der das Rohr (52) gegenüber dem dritten Injektionsrohr mündet.

6. Vorrichtung zur Entnahme von Flüssigkeitsproben in Fläschchen (F), die durch Kapseln (O) verschlossen werden, dadurch gekennzeichnet, daß sie eine Transportvorrichtung (10) nach einem der Ansprüche 1 bis 5 umfaßt, wobei die Fläschchen in den Behältern (36) der Platte angeordnet sind, wobei diese Vorrichtung außerdem einen festen, lateralen Rahmen (40), der eine vertikale Entnahmenadel (58) über Vorrichtungen (64) zum vertikalen Bewegen dieser Nadel zwischen einer hohen Position, die die Rotation der Platte erlaubt, und einer niedrigen Entnahmeposition, in der die Kapsel durch die Nadel durchbohrt wird, trägt, und ein Chromatographieventil (70) umfaßt, das mit einer Probenschleife (72) versehen ist, wobei dieses Ventil eine Position zur Entnahme von Proben einnehmen kann, in der zwei erste Eingänge (6, 5), die jeweils mit der Nadel (58) und einem Venturisaugrohr (86) in Verbindung stehen, miteinander über diese Schleife (72) verbunden werden, und eine Position zum Entleeren der Probe einnehmen kann, in der zwei weitere Eingänge (2, 3) des Ventils, die jeweils mit Ausstoßvorrichtungen (78) für eine entnommene Probe und einem Entleerungsrohr (80) in Verbindung stehen, miteinander über diese Schleife (72) verbunden werden.

7. Vorrichtung nach Anspruch 6, dadurch gekennzeichnet, daß die Ausstoßvorrichtungen für eine entnommene Probe Vorrichtungen (78) umfassen, um gleichzeitig ein bekanntes Volumen von flüssigem Lösungsmittel auszustoßen.

8. Vorrichtung nach Anspruch 7, dadurch gekennzeichnet, daß die Vorrichtungen zum Ausstoßen eines bekannten Volumens von flüssigem Lösungsmittel ein zweites Chromatographieventil (94) umfassen, von dem ein erster Eingang (3), der mit einer Burette (100) in Verbindung steht, mit einem zweiten Eingang (4) verbunden wird, der in einer ersten Stellung des zweiten Ventils mit dem ersten Ventil (70) in Verbindung steht, wobei dieser erste Eingang (3) in einer zweiten Stellung des zweiten Ventils mit einem dritten Eingang (2) verbunden ist, der mit einem Vorrat von flüssigem Lösungsmittel (98) in Verbindung steht.

9. Vorrichtung nach einem der Ansprüche 6 bis 8, dadurch gekennzeichnet, daß die Behälter (36) mit einem gegebenen Abstand auf der Platte (24) verteilt sind und daß mit flüssigen Proben gefüllte Fläschchen (F) und leere Fläschchen (F') abwechselnd in den Behältern angeordnet sind, daß der vertikale Rahmen (40) ebenfalls eine vertikale Reinjektionsnadel (60) trägt, die um den gegebenen Abstand von der Entnahmenadel (58) versetzt ist, wobei diese Reinjektionsnadel über zweite Vorrichtungen (66) zum vertikalen Bewegen dieser Nadel zwischen einer hohen Position, die die Rotation der Platte erlaubt, und einer tiefen Entleerungsposition, in der die Kapsel (O') eines leeren Fläschchens (F') von der Reinjektionsnadel (60) perforiert wird, getragen wird, wobei letztere mit dem Probenentleerungsrohr (80) in Verbindung steht.

10. Vorrichtung nach Anspruch 9, dadurch gekennzeichnet, daß der laterale Rahmen (40) ebenfalls eine vertikale Nadel (62) zur Entnahme einer verdünnten Probe trägt, die um den gegebenen Abstand von der Reinjektionsnadel (60) versetzt ist, wobei diese Nadel zur Entnahme einer verdünnten Probe über dritte Vorrichtungen (68) zum vertikalen Bewegen dieser Nadel zwischen einer hohen Position, die die Rotation der Platte erlaubt, und einer tiefen Entnahmeposition, in der die Kapsel (O') eines mit einer verdünnten Probe gefüllten Fläschchens (F') von der Nadel zur Entnahme einer verdünnten Probe (62) perforiert wird, getragen wird, wobei diese Nadel zur Entnahme einer verdünnten Probe mit einem Probenentnahmerohr (110) in Verbindung steht.

11. Vorrichtung nach Anspruch 10, dadurch gekennzeichnet, daß sie ein drittes Chromatographieventil (102) umfaßt, daß mit einer zweiten Probenschleife (104) versehen ist, wobei dieses Ventil entweder eine Position zur Entnahme einer verdünnten Probe einnimmt, in der zwei erste Eingänge (2, 3), die jeweils mit dem Entnahmerohr (110) und einem zweiten Venturisaugrohr in Verbindung stehen, miteinander über die zweite Probenschleife (104) verbunden werden, oder eine Position zum Entleeren der verdünnten Probe einnimmt, in der zwei weitere Eingänge (S, 6) des dritten Ventils, die jeweils mit einer Injektionsvorrichtung (114) für eine Transferflüssigkeit und einem Analyseapparat (108) in Verbindung stehen, miteinander über die zweite Probenschleife (104) verbunden werden.

## Claims

1. Stepwise advancing rotary conveyor, comprising a fixed base (12) having a horizontal upper face (12a) onto which issues at least one injector (18) communicating with pressurized gas supply means (22), a rotary plate (24) resting by gravity on the upper face of the base and cooperating with said face by means (16, 26a) for centring the plate about a fixed vertical axis, said plate having a circular outer edge (26c, 28b), an upper face provided with receptacles (36) distributed in ring-like manner about said axis and a lower face (26b) defining with the upper face (12a) of the base a central support chamber (30), at least one passage (32) oriented in a direction inclined with respect to a radius passing through said axis being formed in the plate (24) in order to connect the central support chamber and the outer edge of the plate in order to ensure a rotation of the latter when the supply means (22) are actuated, the plate having a high weight such that the rotation is instantaneously stopped when the supply means are stopped, means (38, 42, 44) for the remote detection of the presence of a receptacle (36) in a given angular position about said axis and control means (122, 120) sensitive to signals supplied by the detection means (44) in order to control the stoppage of the supply means (22) when a receptacle (36) is present in said angular position.

2. Conveyor according to claim 1, characterized in that the remote detection means comprise an optical detector (42) integral with the fixed base and reference marks (38) formed on the rotary plate (24) facing said detector in radial planes associated with each of the receptacles.

3. Conveyor according to either of the claims 1 and 2, characterized in that it also comprises means for the remote marking of an initial angular position of the plate, comprising a second optical detector (48) integral with the fixed base and a reference mark (46) formed on the rotary plate (24) facing said second detector in a radial plane passing through one of the receptacles (36).

4. Conveyor according to any one of the claims 1 to 3, characterized in that it also comprises agitating means comprising a second injector (54) integral with the fixed base, pulsed pressurized gas supply means (56) connected to said second injector and at least one pipe (52) formed in plate (24) and having two ends respectively issuing into the bottom of one of the receptacles (36) and in front of the second injector in a given angular position of the plate.

5. Conveyor according to claim 4, characterized in that it also comprises at least one ejection station having a third injector, to which are connected pressurized gas polymers, as well as a discharge chute or tube, whereof one end is placed above one of the receptacles (36) for a given angular position of the plate, in which said pipe (52) issues in front of the third injector.

6. Installation for taking liquid samples from bottles (F) sealed by caps (O), characterized in that it comprises a conveyor (10) according to any one of the claims 1 to 5, the bottles being placed in receptacles (36) of said plate, whereby said installation also incorporates a fixed lateral gantry (40) supporting a vertical sampling needle (58) via means (64) for displacing said needle vertically between an upper position authorizing the rotation of the plate and a lower sampling position, in which the cap is perforated by the needle, and a chromatographic valve (70) equipped with a sampling loop (72), whereby said valve can occupy either a sampling position in which two first inlets (6, 5), respectively communicating with the needle (58Z) and with a suction venturi (86), are interconnected across said loop (72), or a sample discharge position, in which two other inlets (2, 3) of the valve, communicating respectively with means (78) for ejecting a sample taken and with a sample discharge tube (80), are interconnected across said loop (72).

7. Installation according to claim 6, characterized in that the sample ejection means incorporate means (78) for simultaneously ejecting a known liquid diluent volume.

8. Installation according to claim 7, characterized in that the means for ejecting a known liquid diluent volume incorporate a second chromatograhic valve (94), whereof a first inlet (3) communicating with a burette (100) is connected to a second inlet (4) communicating with the first valve (70) in a first position of the second valve, said first inlet (3) being connected to a third inlet (2) communicating with a liquid diluent tank (98) in a second position of the second valve.

9. Installation according to any one of the claims 6 to 8, characterized in that with the receptacles (36) spaced by a given spacing on plate (24) and the bottles (F) filled with liquid samples and the empty bottles (F') being alternately placed in the receptacles, the lateral gantry (40) also supports a vertical reinjection needle (60) displaced by said spacing from the sampling needle (58), said reinjection needle being supported via second means (66) for vertically displacing said needle between an upper position authorizing the rotation of the plate and a lower discharge position in which the cap (O') of an empty bottle (F') is perforated by the reinjection needle (60), the latter communicating with the discharge tube of sample (80).

10. Installation according to claim 9, characterized in that the lateral gantry (40) also supports a vertical diluted sample taking needle (62) displaced by said spacing with respect to the reinjection needle (60), said diluted sample taking needle being supported via third means (68) for displacing said needle vertically between an upper position authorizing the rotation of the plate and a lower sampling position, in which the cap (O') of a bottle (F') filled with diluted sample is perforated by the diluted sample taking needle (62), the latter communicating with a sampling tube (110).

11. Installation according to claim 10, characterized in that it comprises a third chromatographic valve (102) provided with a second sampling loop (104), said valve being able to occupy either a diluted sample taking position, in which two first inlets (2, 3) respectively communicating with the sampling tube (110) and with a second suction venturi are interconnected across the second sampling loop (104), or a diluted pole discharge position, in which two other inlets (5, 6) of the third valve, respectively communicating with a transfer liquid injection means (114) and an analyzer (108) are interconnected across the second loop (104).

FIG. 1

EP 0 286 536 B1

FIG. 2

FIG. 3

FIG. 4